# EUROPÄISCHE PATENTSCHRIFT

(11) **EP 0 442 135 B1**
(45) Veröffentlichungstag und Bekanntmachung des Hinweises auf die Patenterteilung: **23.11.1994**
(21) Anmeldenummer: 90125319.5
(22) Anmeldetag: 22.12.1990
(51) Int. Cl.: A61M 5/14, A61M 39/00

(54) **Vorrichtung zum Zusammenführen mehrerer Infusionen und/oder Injektionen**
Device for bringing together a plurality of infusions and/or injections
Dispositif pour réunir plusieurs infusions et/ou injections

(30) Priorität: 10.02.1990 DE 4004134
(43) Veröffentlichungstag der Anmeldung: 21.08.1991
(73) Patentinhaber: WEX, Roland, D-34212 Melsungen (DE)
(72) Erfinder: WEX, Roland, D-34212 Melsungen (DE)
(74) Vertreter: Quermann, Helmut, Dipl.-Ing.

(56) Entgegenhaltungen:
- EP-A- 0 343 501
- US-A- 4 604 093
- US-A- 4 666 429

## Beschreibung

Die Erfindung betrifft eine Vorrichtung zum Zusammenführen mehrerer Infusionen und/oder Injektionen, mit einem von der Infusions-/Injektionsflüssigkeit durchströmten Gehäuse mit mehreren strahlenförmig angeordneten, jeweils separat verschließbaren Zugängen, sowie mit einem Abgang für die zusammengeführte Flüssigkeit.

Eine derartige Vorrichtung ist aus der DE 35 20 044 A1 bekannt. Mit ihr können Medikament- und/oder Nährlösungen, insbesondere parenterale Nährlösungen verabreicht und/oder hergestellt werden, des weiteren Injektionen vorgenommen werden. Jedem Zugang ist ein nur zum patientenseitigen Auslaß hin öffenbares Rückschlagventil zugeordnet, das insbesondere als Schnabelventil ausgebildet ist.

Die bekannte Vorrichtung weist zwar im Unterschied zu bekannten Hahnbänken, die den Zugängen zugeordnete mechanisch betätigbare Drehhähne aufweisen, den Vorteil auf, daß die Zugänge nicht manuell betätigt werden müssen, nachteilig ist aber, daß die einzelnen Rückschlagventile einen recht hohen Bauaufwand und eine komplizierte Herstellung der Vorrichtung bedingen.

Es ist Aufgabe der vorliegenden Erfindung, eine Vorrichtung der genannten Art zu schaffen, bei der mit einfachen baulichen Mitteln bei einfacher Herstellung eine sichere betätigungsfreie Abdichtung der einzelnen Zugänge gewährleistet ist. Zudem soll die Vorrichtung sowohl für Schwerkraft- als auch Pumpeninfusionen geeignet sein.

Gelöst wird die Aufgabe dadurch, daß das Gehäuse aus einem ersten Gehäuseteil, das die Zugänge aufweist und einem zweiten Gehäuseteil gebildet ist, zwischen denen ein elastisches Dichtelement, das sich zumindest über den Strahlenwinkel der Zugänge erstreckt, im äußeren Bereich des Umfangs gehalten ist, wobei jeder Zugang in einen mit dem Abgang verbundenen Dichtelementöffnungsraum mündet und der dem jeweiligen Öffnungsraum zugeordnete Dichtelementabschnitt gegen den Zugang vorgespannt ist.

Wesentliches Merkmal der vorliegenden Erfindung stellt somit das Dichtelement dar, dem nicht nur die Funktion zukommt, die beiden Gehäuseteile gegeneinander abzudichten, sondern gleichfalls Ventilfunktion. Das einzige Dichtelement ist dort, wo sich keine Öffnungsräume befinden, zwischen den beiden Gehäuseteilen eingeklemmt und damit nur im Bereich der Öffnungsräume von den Zugängen weg entgegen der Vorspannungskraft nachgiebig. Die Vorspannung selbst kann beliebig auf die jeweiligen Dichtelementabschnitte aufgebracht werden, beispielsweise indem das Dichtelement im Bereich der Dichtelementabschnitte aufgrund einer Verformung unter einer Eigenspannung steht oder zwischen dem jeweiligen Dichtelementabschnitt und dem dem jeweiligen Zugang abgewandten Gehäuseteil zwischen dem Dichtelementabschnitt und dem Gehäuseteil ein elastisches Element angeordnet ist, das beim Zuführen einer Flüssigkeit, sei es im Wege einer Infusion oder einer Injektion, vom von der Öffnung abhebenden Dichtelementabschnitt zusammengedrückt wird. Das Dichtelement besteht beispielsweise aus Silicon oder Gummi. Es kann mit beidseits des jeweiligen Zuganges angeordneten, in Durchströmrichtung orientierten Schlitzen versehen sein, die zwischen sich einen Dichtlappen bilden, um so bei verminderter Vorspannung des Dichtelementabschnittes dessen Öffnungsbewegung zu erleichtern.

Die Abdichtung der Zugänge mittels identisch wirkender Dichtelementabschnitte stellt sicher, daß bei gleichen Zuflußbedingungen alle Zugänge die gleiche Förderleistung haben. Des weiteren sind wegen der vorgespannten Dichtelementabschnitte alle Zuflüsse rückflußgesichert. In diesem Zusammenhang kommt der strahlenförmigen Anordnung der Zuflüsse besondere Bedeutung zu, die nicht nur die Zusammenführung mehrerer Infusionen und/oder Injektionen auf kleinstem Raum gestattet und eine ergonomisch günstige Anordnung der Zuflüsse gewährleistet, sondern auch eine besonders einfache bauliche Gestaltung des Gehäuses und des Dichtelementes. So sieht eine bevorzugte Ausführungsform der Erfindung vor, daß das Dichtelement konzentrisch zum Abgang angeordnet ist und mit seinem inneren Umfang beabstandet zur Innenbegrenzung des jeweiligen Öffnungsraumes endet. Der Vorteil dieser Ausgestaltung ist darin zu sehen, daß die Flüssigkeit von den Zugängen ausgehend auf direktem Weg radial nach innen zum Abgang strömt und die Strömungsführung aufgrund der kurzen Durchlaufwege optimal ist, bei gleichzeitig intensiver Vermischung der Flüssigkeiten.

Es wird als besonders vorteilhaft angesehen, wenn das erste Gehäuseteil im Bereich jedes Zugang eine vom zweiten Gehäuseteil weggerichtete Wölbung aufweist, die mit einer Zugangsöffnung versehen ist, sowie der jeweilige Dichtelementabschnitt im Bereich der jeweiligen Wölbung mit einer im wesentlichen entsprechenden Wölbung versehen ist. Die Abdichtung der einzelnen Zugangsöffnungen erfolgt somit im Bereich der zugeordneten Wölbungen durch Zusammenwirken der gewölbten Bereiche der Dichtelementabschnitte mit den gewölbten Bereichen des ersten Gehäuseteils. Die eingespannte Lagerung des Dichtelementes in unmittelbarer Nachbarschaft zu deren gewölbten Bereichen bewirkt, daß die gewölbten Dichtelementabschnitte beim Zuführen von Flüssigkeit von deren Scheitelpunkt ausgehend von der Zugangsöffnung abheben und nach Ende der Flüssigkeitszuführung sofort ihre gleichmäßig gewölbte Stellung wieder einnehmen, bei der die jeweilige Zugangsöffnung verschlossen wird. Vorteilhaft ist es, wenn auch das zweite Gehäuseteil im Bereich jedes Zuganges eine auf das erste Gehäuseteil gerichtete Wölbung aufweist, die bei jeweiligem, geschlossenem Zufluß einen Spalt zum korrespondierenden Dichtelementabschnitt freiläßt. Es kann dieser gewölbte Dichtelementabschnitt damit beim Öffnen des Zuganges vom ersten Gehäuseteil abheben, jedoch nur so weit, bis es am zweiten Gehäuseteil anliegt, womit dieses ein Überschnappen des Dichtelementabschnittes verhindert. Die beschriebene Ausbildung beinhaltet zudem den Vorteil, daß sie nur eine eindeutige Montage des Dichtelementes ermöglicht. Vorteilhaft stellen sich die Gehäuseteile und die Dichtelementabschnitte in Form einer Halbschale dar. Um eine eindeutige Vorspannung der Dichtelementabschnitte zu gewährleisten, sollten diese bei jeweiligem, nicht vorgespanntem Dichtelementabschnitt, somit in unmontiertem Zustand, eine solche Wölbung aufweisen, die geringer ist als die des ersten Gehäuseteils. Es reicht eine geringfügig geringere Krümmung der Dichtelementabschnitte aus, so daß diese beim Zusammenfügen der beiden Gehäuseteile über deren jeweilige gesamte Wölbung in die jeweils zugeordnete Wölbung des ersten Gehäuseteiles eingedrückt wird.

Eine besondere Ausgestaltung des Dichtelementes sieht vor, daß es am äußeren Umfang einen axialen Ansatz aufweist und zwischen äußeren radialen Bereichen der Gehäuseteile sowie zwischen dem axialen Ansatz zugeordneten axialen Bereichen der Gehäuseteile geklemmt ist. Das Gehäuse ist vorteilhaft im wesentlichen flach ausgebildet und es münden die Dichtelementöffnungsräume radial in einen durch die beiden Gehäuseteile begrenzten flachen Flüssigkeitssammelraum, der zentrisch angeordnet den zum Patienten führenden Abgang aufweist, der bevorzugt axial angeordnet ist. Bei einer flachen Ausbildung des Gehäuses enden die jeweiligen Dichtelementabschnitte zweckmäßig radial innen beabstandet zu einer, mit dem ersten Gehäuseteil verbundenen Überströmwandung, die zwischen dem jeweiligen Öffnungsraum und dem Flüssigkeitssammelraum angeordnet ist. Die Überströmwandung stellt sicher, daß beim Zuführen von Flüssigkeit an anderen Zugängen der Vorrichtung die Flüssigkeit nicht unter den Dichtelementabschnitt desjenigen Zuganges, bei dem keine Flüssigkeitszuführung erfolgt, gelangen kann und überdies ein Druckaufbau auf den lippenartigen Dichtelementabschnitt stattfindet.

Um den patientenseitigen Abgang gegen den Durchlaß von Mikropartikeln zu sichern, ist vorteilhaft eine hydrophile Membran im Strömungsweg der Flüssigkeit zwischen Dichtelementoffnungsräumen und Abgang im Flüssigkeitssammelraum angeordnet. Ferner kann zum Zwecke der Zwangsentlüftung des patientenseitigen Abflusses das Gehäuse mit Entlüftungsbohrungen versehen sein, denen eine hydrophobe Membran zugeordnet ist. Es wird als zweckmäßig angesehen, wenn das erste Gehäuseteil das Unterteil des Gehäuses und das zweite Gehäuseteil das Oberteil des Gehäuses darstellt, in diesem Fall weist das Gehäuseoberteil die Entlüftungsbohrungen auf. Konzentrisch zur hydrophilen und/oder hydrophoben Membran ist bevorzugt das Dichtelement angeordnet.

Um die Handhabung der Vorrichtung möglichst einfach zu gestalten und um diese auch dauerhaft positionieren zu können, was insbesondere bei Infusionen bedeutungsvoll ist, ist vorgesehen, daß das Gehäuse aus einem Halbkreisabschnitt mit den sich über den Halbkreissektor erstreckenden radialen Zugängen und einer sich an den Basisbereich des Halbkreisabschnittes anschließenden, in der Ebene des Halbkreisabschnittes orientierten Halterungsplatte gebildet ist. Die Halterungsplatte ermöglicht es, die Vorrichtung beispielsweise an einem Stativ einzuspannen, wobei anzustreben ist, daß die Zugänge horizontal und der Abgang vertikal nach unten gerichtet sind, was das Handling wesentlich vereinfacht und die Abknickungsgefahr für zu den Zuflüssen führende Schläuche verringert.

In Weiterbildung kann schließlich vorgesehen sein, die Zugänge und den Abgang kontaminations- und luftdicht zu schützen, mittels einer Kappe (Luer-Lock) und es können den Infusionsleitungen separate Klemmen zugeordnet sein, die dem Zweck dienen, die Infusionsleitungen voll abzuklemmen oder den Durchfluß in den Infusionsleitungen zu dosieren. Daneben können mehrere Vorrichtungen in Parallel- oder Reinschaltung miteinander gekoppelt werden. Zur Unterscheidung der einzelnen Zugänge werden diese zweckmäßig farbig gekennzeichnet.

Weitere Merkmale der Erfindung sind in der Beschreibung der Figuren und in den Unteransprüchen dargestellt, wobei bemerkt wird, daß alle Einzelmerkmale und alle Kombinationen von Einzelmerkmalen weitere erfinderische Ausgestaltungen darstellen.

In den Figuren ist die Erfindung an einer Ausführungsform beispielsweise dargestellt, ohne auf diese beschränkt zu sein. Es stellt dar:
- Figur 1: eine Draufsicht der erfindungsgemäßen Vorrichtung (Ansicht X gemäß Figur 2),
- Figur 2: einen Schnitt durch die Vorrichtung gemäß der Linie A-B in Figur 1 und
- Figur 3: einen Schnitt durch die Vorrichtung im Bereich eines Zuganges gemäß der Linie C-D in Figur 2.
- Figur 4: eine Unteransicht eines abgewandelt gestalteten Dichtelementes, in vergrößerter Darstellung.

Die in den Figuren gezeigte Vorrichtung stellt einen Sammler für Infusionen und/oder Injektionen dar, der sowohl für Schwerkraftals auch Pumpeninfusionen geeignet ist. Er weist ein Gehäuse 1 mit fünf um einen Winkel von jeweils 45° versetzten Zugängen 2 auf, die auf die später noch zu beschreibende Betriebsposition der Vorrichtung bezogen waagerecht angeordnet sind, ferner einen senkrecht nach unten orientierten Abgang 3.

Im Detail besteht das Gehäuse 1 aus einem im wesentlichen flachen, horizontal orientierten Gehäuseunterteil 1a und einem flachen Gehäuseoberteil 1b. Das Gehäuseunterteil 1a weist einen sich im wesentlichen über einen Halbkreis erstreckenden, ebenen Mittelabschnitt 4 auf, an den sich nach unten als Abgang 3 ein Stutzen 5 mit Luer-Lock-Kegel anschließt. Seitlich wird der Mittelabschnitt 4 durch einen sich senkrecht hierzu vom Stutzen weg erstreckenden umlaufenden Rand 6 begrenzt. Im Bereich der Zugänge 2 ist das Gehäuseunterteil 1a nach unten gekröpft ausgebildet, jeder Zugang 2 stellt sich als Stutzen 7 mit Luer-Lock-Kegel dar, der einen Winkelkanal aufweist, der vom Stutzen 7 ausgehend einen radialen Kanalabschnitt 8 und einen sich hieran anschließenden axial nach oben gerichteten Kanalabschnitt 9 aufweist. Radial innerhalb der Austrittsöffnung des Kanalabschnittes 9 ist das Gehäuseunterteil 1a mit einem sich geringfügig nach oben erstreckenden Steg 10 versehen, der sich entsprechend der Anordnung der Zugänge 2 über einen Halbkreis erstreckt und im Bereich seiner beiden Enden um einen geraden Abschnitt verlängert ist. Radial außerhalb des Steges 10 ist, wie insbesondere der Darstellung der Figur 3 zu entnehmen ist, das Gehäuseunterteil 1a als nach unten gewölbte Halbschale 15 ausgebildet, die in Blickrichtung nach radial außen betrachtet nach unten geneigt ist.

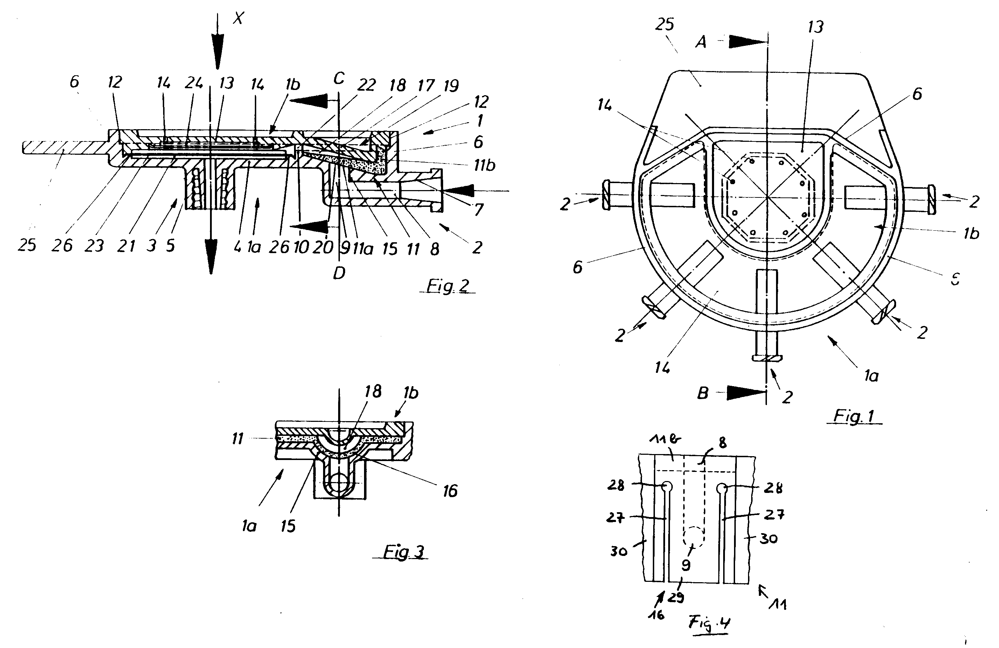

Zwischen den umlaufenden Rand 6 und den Steg 10 ist ein elastisches Dichtelement 11 eingesetzt, das beispielsweise aus Silicon besteht. Dieses erstreckt sich, wie in der Figur 1 mit gestrichelten Linien verdeutlicht, entsprechend der Anordnung der Zugänge 2 über einen Halbkreis und endet innen in geringfügigem Abstand von dem Steg 10. Das Dichtelement ist im Bereich seines dem Steg 10 zugewandten Endes relativ schmal ausgebildet, in Art einer Dichtlippe und es erweitert sich der der Dichtlippe zugeordnete Dichtungsabschnitt 11a des Dichtelementes nach außen hin und setzt sich senkrecht hierzu in einem nach oben gerichteten Lagerabschnitt 11b fort. Im Bereich der Zugänge 2, was in den Figuren 2 und 3 verdeutlicht ist, ist der Dichtungsabschnitt 11a des Dichtelementes 11 entsprechend der Halbschale 15 des Gehäuseunterteiles 1a gestaltet, so daß dort die Halbschale 16 des Dichtelementes 11 an der Halbschale 15 des Gehäuseunterteiles 1a anliegt. Im Detail ist die Krümmung der Halbschale 16 geringfügig geringer bemessen als die der Halbschale 15.

Das Gehäuseoberteil 1b ist entsprechend der durch den umlaufenden Rand 6 des Gehäuseunterteils 1a vorgegebenen Form gestaltet, das heißt es ist im wesentlichen halbkreisförmig ausgebildet, mit einem sich hieran anschließenden trapezförmigen Ansatz. Es läßt sich in das Gehäuseunterteil 1a einpassen, wobei dann der äußere Rand 12 des Gehäuseoberteils 1b dichtend mit der Innenkontur des umlaufenden Randes 6 zusammenwirkt. Parallel beabstandet zum Mittelabschnitt 4 des Gehäuseunterteiles 1a ist das Gehäuseoberteil 1b mit einem zentralen Abschnitt 13 versehen, der Entlüftungsbohrungen 14 aufweist. An den zentralen Abschnitt 13 schließt sich ein Randabschnitt 19 an, der im Bereich der Zugänge 2, wie insbesondere in Figur 3 verdeutlicht, wiederum als Halbschale 17 ausgebildet ist. Die einander zugewandten Konturen der Halbschalen 15 und 17 verlaufen parallel zueinander und bilden zwischen sich einen Dichtelementöffnungsraum 18. Bei zusammengesetztem Gehäuse 1 preßt insbesondere das im Bereich der Zugänge 2 gebildete Knie des Randabschnittes 19 das Knie zwischen dem Dichtabschnitt 11a und dem Lagerabschnitt 11b des Dichtelementes 11 gegen die Halbschale 15, des weiteren wird allgemein das Dichtelement 11 zwischen den jeweils benachbarten Zugängen 2 zwischen dem Gehäuseoberteil 1b und dem Gehäuseunterteil zusammengepreßt. Dies führt, unterstützt durch die zusätzliche Vorspannung infolge der unterschiedlichen Wölbungen der Halbschale 16 des Dichtelementes 11 und der Halbschale 15 des Gehäuseunterteiles 1a zu einem sicheren Verschließen der jeweiligen Öffnung 20 der Zugänge 2.

Zwischen dem zentralen Abschnitt 13 des Gehäuseoberteiles 1b und dem Mittelabschnitt 4 des Gehäuseunterteils 1a ist ein Flüssigkeitssammelraum 21 gebildet, der mit jedem Dichtelementöffnungsraum 18 des zugeordneten Zuganges 2 über eine stegseitige Überströmöffnung 22 in Strömungsverbindung steht. Zwischen den Überströmöffnungen 22 und dem Stutzen 5 ist im Gehäuseunterteil eine hydrophile Membran 23 angeordnet, andererseits im Gehäuseoberteil 1b vor den Entlüftungsbohrungen 14 eine hydrophobe Membran 24. Konzentrisch zu den Membranen 23 und 24 ist das Dichtelement 11 angeordnet. Das Gehäuse 1 ist bezüglich der Linie A-B symmetrisch ausgebildet und es weist das Gehäuseunterteil 1a auf seiner dem in der Symmetrieebene angeordneten Zugang 2 abgewandten Seite eine Halteplatte 25 auf.

Das Gehäuse besteht beispielsweise aus einem thermoplastisch verformbaren, durchsichtigen Kunststoff, insbesondere PVC, mit dem dem Mittelabschnitt 4 des Gehäuseunterteils 1a zugeordneten, erhabenen Ringabschnitt 26 ist die hydrophile Membran 23 beispielsweise verschweißt oder wird zwischen diesem Ringabschnitt und dem Gehäuseoberteil 1b eingeklemmt, entsprechend mit einem erhabenen Bereich des zentralen Abschnittes 13 des Gehäuseoberteiles 1b die hydrophobe Membran 24 verschweißt. Nach dem Einlegen des Dichtungselementes 11 in das Gehäuseunterteil 1a und dem Einpassen des Gehäuseoberteiles 1b in das Gehäuseunterteil 1a werden beide Gehäuseteile zweckmäßig gleichfalls miteinander verschweißt. Vor dem Gebrauch der erfindungsgemäßen Vorrichtung sind die Zugänge 2 und der Abgang 3 vorteilhaft mittels Stopfen verschlossen, überdies sind die Zugänge vorteilhaft farbig gekennzeichnet.

Vor dem Gebrauch der Vorrichtung wird die Halteplatte 25 bei horizontaler Orientierung des Gehäuses 1 an einem Ständer fixiert, ferner über eine Schlauchverbindung der Abgang 3 mit einem Patienten verbunden und entsprechend der Anzahl der Infusionen die Infusionsschläuche auf die Zugänge 2 geschraubt. Im Gebrauch wird bei einem ausreichenden Druck in der Infusionsleitung der Scheitelbereich des Dichtungsabschnittes 11a des jeweiligen Dichtelementes 11 von der Öffnung 20 und dem zwischen der Öffnung 20 und dem Steg 10 zugewandten Bereich des Gehäuseunterteils 1a wegbewegt, womit eine geringfügig gegenläufige Einwölbung der Halbschale 16 entsteht und die Flüssigkeit radial nach Innen über die Überströmöffnung 24 in den Flüssigkeitssammelraum 21 gelangen kann und von dort zum Abgang 3. Wird die Infusion abgeklemmt, führt die Druckverminderung im zugeordneten Zugang 2 zu einem unverzüglichen Schließen des Dichtelementes aufgrund der Vorspannung. Ein Ausströmen der Flüssigkeit aus anderen Zugängen 2, die aktuell mit keiner Infusion oder Injektion beaufschlagt werden, ist ausgeschlossen, da der Flüssigkeitsdruck auf die der Öffnung 20 abgewandte Seite des Dichtelementes 11 wirkt und hierdurch sogar einen erhöhten Schließdruck bewirkt. Vorteilhaft ist die Anordnung des Steges 10 mit der Überstromöffnung 22, die nach dem Überströmprinzip den Druckaufbau im Bereich des inneren lippenartigen Endes des Dichtelementes fördert.

Figur 4 zeigt eine gegenüber der beschriebenen Gestaltung des Dichtelementes 11 abgewandelte Ausführungsform. Bei dieser ist die zwischen den ebenen Bereichen 30 des Dichtelementes 11 angeordnete Halbschale 16 des Dichtelementes 11 auf beiden Seiten des Kanalabschnittes 9 beabstandet zu diesem mit parallel zueinander und parallel zum radialen Kanalabschnitt 8 angeordneten Schlitzen 27 versehen, wobei der jeweilige Schlitz 27 benachbart des Steges 10 offen ist und das andere Schlitzende als Bohrung 28 ausgebildet ist. Der hierdurch gebildete Dichtlappen 29 dichtet den Kanalabschnitt 9 unter verminderter Vorspannung ab.

### Bezugszeichenliste

- 1: Gehäuse
- 1a: Gehäuseunterteil
- 1b: Gehäuseoberteil
- 2: Zugang
- 3: Abgang
- 4: Mittelabschnitt
- 5: Stutzen
- 6: umlaufender Rand
- 7: Stutzen
- 8: radialer Kanalabschnitt
- 9: axialer Kanalabschnitt
- 10: Steg
- 11: Dichtelement
- 11a: Dichtungsabschnitt
- 11b: Lagerabschnitt
- 12: äußerer Rand
- 13: zentraler Abschnitt
- 14: Entlüftungsbohrung
- 15: Halbschale
- 16: Halbschale
- 17: Halbschale
- 18: Dichtelementöffnungsraum
- 19: Randabschnitt
- 20: Öffnung
- 21: Flüssigkeitssammelraum
- 22: Überströmöffnung
- 23: hydrophile Membran
- 24: hydrophobe Membran
- 25: Halteplatte
- 26: Ringabschnitt
- 27: Schlitz
- 28: Bohrung
- 29: Dichtlappen
- 30: ebener Bereich

## Patentansprüche

1. Vorrichtung zum Zusammenführen mehrerer Infusionen und/oder Injektionen, mit einem von der Infusions-/Injektionsflüssigkeit durchströmten Gehäuse mit mehreren strahlenförmig angeordneten, jeweils separat verschließbaren Zugängen, sowie mit einem Abgang für die zusammengeführte Flüssigkeit, **dadurch gekennzeichnet**, daß das Gehäuse (1) aus einem ersten Gehäuseteil (1a), das die Zugänge (2) aufweist und einem zweiten Gehäuseteil (1b) gebildet ist, zwischen denen ein elastisches Dichtelement (11), das sich zumindest über den Strahlenwinkel der Zugänge (2) erstreckt, im äußeren Bereich des Umfangs gehalten ist, wobei jeder Zugang (2) in einen mit dem Abgang (3) verbundenen Dichtelementöffnungsraum (18) mündet und der dem jeweiligen Öffnungsraum (18) zugeordnete Dichtelementabschnitt (11a) gegen den Zugang (2) vorgespannt ist.

2. Vorrichtung nach Anspruch 1, **dadurch gekennzeichnet**, daß das Dichtelement (11) konzentrisch zum Abgang (3) angeordnet ist und mit seinem inneren Umfang beabstandet zur Innenbegrenzung des jeweiligen Öffnungsraumes (18) endet.

3. Vorrichtung nach Anspruch 1 oder 2, **dadurch gekennzeichnet**, daß das erste Gehäuseteil (1a) im Bereich jedes Zugangs (2) eine vom zweiten Gehäuseteil (1b) weggerichte Wölbung (15) aufweist, die eine Öffnung (20) des Zugangs (2) aufweist, sowie der jeweilige Dichtelementabschnitt (11a) im Bereich der jeweiligen Wölbung (15) mit einer im wesentlichen entsprechenden Wölbung (16) versehen ist.

4. Vorrichtung nach Anspruch 3, **dadurch gekennzeichnet**, daß bei nicht vorgespanntem Dichtelementabschnitt (11a) dessen der Wölbung (15) des ersten Gehäuseteils (1a) zugeordnete Wölbung (16) eine geringere Krümmung aufweist als die der Wölbung des Gehäuseteils (1a).

5. Vorrichtung nach einem der Ansprüche 1 bis 4, **dadurch gekennzeichnet**, daß das Dichtelement (11) am äußeren Umfang einen axialen Ansatz (11b) aufweist und zwischen äußeren radialen Bereichen der Gehäuseteile (1a, 1b) sowie zwischen dem axialen Ansatz (11b) zugeordneten axialen Bereichen der Gehäuseteile (1a, 1b) geklemmt ist.

6. Vorrichtung nach einem der Ansprüche 1 bis 5, **dadurch gekennzeichnet**, daß das Gehäuse (1) im wesentlichen flach ausgebildet ist und die Dichtelementöffnungsräume (18) radial in einen durch die beiden Gehäuseteile (1a, 1b) begrenzten flachen Flüssigkeitssammelraum (21) münden, der zentrisch angeordnet den Abgang (3) aufweist, der axial angeordnet ist.

7. Vorrichtung nach Anspruch 6, **dadurch gekennzeichnet**, daß der jeweilige Dichtelementabschnitt (11a) radial innen beabstandet zu einer Bestandteil des ersten Gehäuseteiles (1a) bildenden Überströmwandung (10) endet, die zwischen dem jeweiligen Öffnungsraum (18) und dem Flüssigkeitssammelraum (21) angeordnet ist.

8. Vorrichtung nach Anspruch 6 oder 7, **dadurch gekennzeichnet**, daß eine hydrophile Membran (23) im Strömungsweg der Flüssigkeit zwischen den Dichtelementsöffnungsräumen (18) und dem Abgang (3) im Flüssigkeitssammelraum (21) angeordnet ist.

9. Vorrichtung nach einem der Ansprüche 1 bis 8, **dadurch gekennzeichnet**, daß das erste Gehäuseteil (1a) das Unterteil des Gehäuses (1) und das zweite Gehäuseteil (1b) das Oberteil des Gehäuses (1) darstellt.

10. Vorrichtung nach Anspruch 9, **dadurch gekennzeichnet**, daß das Gehäuseoberteil (1b) Entlüftungsbohrungen (14) aufweist, denen eine hydrophobe Membran (24) zugeordnet ist.

11. Vorrichtung nach einem der Ansprüche 1 bis 10, **dadurch gekennzeichnet**, daß das Gehäuse (1) aus einem Halbkreisabschnitt mit den sich über den Halbkreissektor erstreckenden radialen Zugängen (2) und einer sich an den Basisbereich des Halbkreisabschnittes anschließenden, bevorzugt in dessen Ebene orientierten Halterungsplatte (25) gebildet ist.

12. Vorrichtung nach einem der Ansprüche 1 bis 11, **dadurch gekennzeichnet**, daß das Dichtelement (11) mit beidseitig des jeweiligen Zuganges (9) angeordneten, in Durchströmrichtung orientierten Schlitzen (27) versehen ist, die zwischen sich einen Dichtlappen (29) bilden.

## Claims

1. Device for bringing together a plurality of infusions and/or injections, with a housing through which the infusion/injection liquid flows and which has a plurality of accesses arranged in a radiating pattern and in each case capable of being closed separately, and with an outlet for the liquid which has been brought together, characterized in that the housing (1) consists of a first housing part (1a), which has the accesses (2), and of a second housing part (1b), between which parts an elastic sealing element (11), which extends at least over the radiating angle of the accesses (2), is held in the outer area of the perimeter, each access (2) opening into a sealing element opening chamber (18) connected to the outlet (3), and the sealing element section (11a) assigned to the respective opening chamber (18) being pretensioned against the access (2).

2. Device according to Claim 1, characterized in that the sealing element (11) is arranged concentrically with respect to the outlet (3) and ends with its inner perimeter at a distance from the inner limit of the respective opening chamber (18).

3. Device according to Claim 1 or 2, characterized in that the first housing part (1a) has, in the area of each access (2), a bulge (15) which is directed away from the second housing part (1b) and which has an opening (20) of the access (2), and the respective sealing element section (11a) is provided in the area of the respective bulge (15) with an essentially corresponding bulge (16).

4. Device according to Claim 3, characterized in that, when the sealing element section (11a) is not pretensioned, its bulge (16) assigned to the bulge (15) of the first housing part (1a) has a lesser curvature than that of the bulge of the housing part (1a).

5. Device according to one of Claims 1 to 4, characterized in that the sealing element (11) has an axial shoulder (11b) on the outer perimeter and is clamped between outer radial areas of the housing parts (1a, 1b) and also between axial areas of the housing parts (1a, 1b) assigned to the axial shoulder (11b).

6. Device according to one of Claims 1 to 5, characterized in that the housing (1) is designed essentially flat, and the sealing element opening chambers (18) open radially into a flat liquid-collection chamber (21) which is delimited by the two housing parts (1a, 1b) and which has, in centric arrangement, the outlet (3) which is axially arranged.

7. Device according to Claim 6, characterized in that the respective sealing element section (11a) ends radially inwards at a distance from an overflow wall (10) which is a component of the first housing part (1a) and which is arranged between the respective opening chamber (18) and the liquid-collection chamber (21).

8. Device according to Claim 6 or 7, characterized in that a hydrophilic membrane (23) is arranged in the flow path of the liquid between the sealing element opening chambers (18) and the outlet (3) in the liquid-collection chamber (21).

9. Device according to one of Claims 1 to 8, characterized in that the first housing part (1a) represents the lower part of the housing (1), and the second housing part (1b) represents the upper part of the housing (1).

10. Device according to Claim 9, characterized in that the housing upper part (1b) has ventilation bores (14) to which a hydrophobic membrane (24) is assigned.

11. Device according to one of Claims 1 to 10, characterized in that the housing (1) consists of a semicircle section with the radial accesses (2) extending over the semicircle sector and of a mounting plate (25) which is connected to the base area of the semicircle section and is preferably orientated in the plane thereof.

12. Device according to one of Claims 1 to 11, characterized in that the sealing element (11) is provided with slots (27) which are arranged on both sides of the respective access (9), are orientated in the direction of flow and form between them a sealing tab (29).

## Revendications

1. Dispositif pour réunir plusieurs infusions et/ou injection comprenant un boîtier traversé par le liquide d'infusion/injection, et ayant plusieurs entrées disposées de manière radiale qui peuvent être fermées séparément ainsi qu'une sortie pour le liquide réuni, dispositif caractérisé en ce que le boîtier (1) se compose d'une première partie (1a) comportant des entrées (2) et d'une seconde partie (1b) reliée à la première par un élément d'étanchéité élastique (11) qui occupe au moins l'arc angulaire des entrées (2), en étant maintenu dans la zone extérieure de la périphérie, chaque entrée (2) débouchant dans une chambre d'élément d'étanchéité (18) relié à la sortie (3), le segment (11a) de l'élément d'étanchéité associé à la chambre d'ouverture respective (18) étant précontraint contre l'entrée (2).

2. Dispositif selon la revendication 1, caractérisé en ce que l'élément d'étanchéité (11) est concentrique à la sortie (3) et sa périphérie intérieure s'arrête à distance de la limite intérieure de la chambre d'ouverture (18) respective.

3. Dispositif selon la revendication 1 ou 2, caractérisé en ce que la première partie (1a) du boîtier comporte dans la zone de chaque entrée (2), une partie bombée (15) écartée de la seconde partie de boîtier (1b) et qui présente une ouverture (20) pour l'entrée (2) et le segment d'élément d'étanchéité (11a) respectif est muni au niveau de chaque partie bombée (15) d'une partie (16) bombée essentiellement de manière correspondante.

4. Dispositif selon la revendication 3, caractérisé en ce que pour un segment d'élément d'étanchéité (1a) non précontraint, dont la partie bombée (15) est associée à la partie bombée (16) de la première partie de boîtier (1a) présente une courbure plus faible que la partie bombée de la partie de boîtier (1a).

5. Dispositif selon l'une des revendications 1 à 4, caractérisé en ce que l'élément d'étanchéité (11) présente à la périphérie extérieure un prolongement axial (11b) et est pincé entre les zones radiales extérieures des parties de boîtier (1a, 1b) ainsi qu'entre les zones des parties de boîtier (1a, 1b) axiales, associées au prolongement axial (11b).

6. Dispositif selon l'une des revendications 1 à 5, caractérisé en ce que le boîtier (1) est essentiellement plat et les chambres d'ouverture (18) de l'élément d'étanchéité débouchent radialement dans une chambre collectrice de liquide (21), plate, délimitée par les deux parties de boîtier (1a, 1b), chambre qui comporte en son centre la sortie (3) disposée axialement.

7. Dispositif selon la revendication 6, caractérisé en ce que le segment d'élément d'étanchéité (11a) respectif est écarté radialement vers l'intérieur par rapport à une paroi de débordement (10) qui appartient à la première partie de boîtier (1a) et qui se trouve entre la chambre d'ouverture (18) respective et la chambre collectrice de liquide (21).

8. Dispositif selon la revendication 6 ou 7, caractérisé en ce qu'une membrane hydrophile (23) est prévue dans le chemin d'écoulement du liquide entre les chambres d'ouverture (18) de l'élément d'étanchéité et la sortie (3) de la chambre collectrice de liquide (21).

9. Dispositif selon l'une des revendications 1 à 8, caractérisé en ce que la première partie de boîtier (1a) est la partie inférieure du boîtier (1) et la seconde partie (1b) est la partie supérieure du boîtier (1).

10. Dispositif selon la revendication 9, caractérisé en ce que la partie supérieure (1b) du boîtier comporte des orifices d'évacuation d'air (14) dans lesquels se trouve une membrane hydrophobe (24).

11. Dispositif selon l'une des revendications 1 à 10, caractérisé en ce que le boîtier (1) se compose d'un segment de demi-cercle relié à des entrées (2), radiales, s'étendant sur le secteur en forme de demi-cercle et d'une plaque de fixation (25) adjacente à la zone de base du segment en forme de demi-cercle et qui est de préférence orientée dans son plan.

12. Dispositif selon l'une des revendications 1 à 11, caractérisé en ce que l'élément d'étanchéité (11) comporte des fentes (27) prévues de part et d'autre de l'entrée respective (9), et qui sont orientées dans la direction de passage, ces fentes forment entre elles une patte d'étanchéité (29).
